# EUROPEAN PATENT APPLICATION

(11) **EP 4 533 975 A1**
(43) Date of publication of application: **09.04.2025**
(21) Application number: 23201181.7
(22) Date of filing: 02.10.2023
(51) Int. Cl.: A44C 5/00, A44C 5/02, G08B 21/02, G08B 21/22

(54) **ELECTRONIC MONITORING BRACELET**

(71) Applicant: Geosatis SA, 2340 Le Noirmont (CH)
(72) Inventor: LISON GOMEZ, Alvaro, 2340 Le Noirmont (CH); PRAPLAN, Vincent, 2340 Le Noirmont (CH); Dustour, Jonathan, 2340 Le Noirmont (CH); Perrenoud, Nicolas, 2340 Le Noirmont (CH)
(74) Representative: Bugnion Genève

(57) **Abstract**

Electronic monitoring bracelet (B) comprising a casing configured to surround an ankle or a wrist of a wearer, an electronic unit housed in the casing and a battery housed in the casing, the casing is made of a first part (4) and a second part (6) connected to each other by at least one mechanical connection, said mechanical connection comprising two pins (10, 12) extending from a first end face of the first part and two housings (14, 16) made opening in a first end face of the second part (6), each housing (14, 16) being configured to house one pin (10, 12), the mechanical connection also comprising locking means for retaining each pin (10, 12) in its housing, said locking means comprising at least one locking plate movable transversely with respect to the housing axis and a drive unit for moving the locking plate, the locking plate and both pins (10, 12) being shaped in such manner that, in a locking state, the pins (10, 12) are retained in their housing.

## Description

### TECHNICAL FIELD AND PRIOR ART

The present invention relates to an electronic monitoring bracelet.

Electronic monitoring bracelet are used for monitoring the displacements of a person, for example within the scope of judicial oversight.

Certain persons suspected of having committed an offence awaiting judgment, or having committed an offence and being on parole, may be forced to wear an electronic monitoring bracelet so that the monitoring authority may localize the person and monitor his/her displacements at any time. Such bracelets contain a satellite localization system (called "GNSS", acronym for Global Navigation Satellite System) such as for example GPS (Global Positioning System), a telecommunications system for transmitting data to a monitoring/control central unit and a system for detecting integrity of the bracelet in order to be able to detect whether the bracelet has been taken off or is faulty.

For example, document EP2795588 describes such an electronic monitoring bracelet. The bracelet has a rigid ring-shaped shell in which the electronic systems are housed. The internal energy source is provided by rechargeable batteries located inside the casing of the bracelet.

The body of the bracelet comprises two portions which are mechanically connected. The mechanical connection comprises pins on one portion which cooperates with housings in the other portion, and plungers retain the pins in the housings in a close state. An electromagnet is controlled to move the plungers, which liberate the pins that can slide out of the housings. The two portions of the bracelet can then be separated.

This bracelet performs satisfactorily. But there is a need for a mechanical connection which does not need electromagnet.

### SUMMARY OF THE INVENTION

It is therefore one goal of the present invention to offer an electronic monitoring bracelet with a new mechanical connection.

The above-recited goal is fulfilled by an electronic monitoring bracelet comprising at least two portions and mechanical connection connecting the portions, at least one portion comprising at least one pin or tube and the other portion comprising at least one housing configured to house the pin, locking means for locking the pin in the housing. Such locking means comprising a movable locking plate in a direction perpendicular to the pin and a drive unit for moving the plate between a locking position and an unlocking position. The pin has shape configured to cooperate with an edge in the locking plate, which forms an abutment for the pin. The movable plate is configured to have at least a locking position in which the locking plate forms an abutment for the pin and prevents it from escaping from the housing and then preventing both parts from being separated from each other, and an unlocking position in which the pin can escape from the housing. The drive unit is controlled to move the plate at least between the locking position and the unlocking position.

Preferably, the drive unit comprises an electrical motor and a cam, the cam cooperating with a cam edge made in the locking plate.

In a preferred embodiment, the mechanical connection comprises at least two pins and two housings and the plate comprises two recesses, each one being configured to cooperate with one pin.

The main subject-matter is then an electronic monitoring bracelet comprising a casing configured to surround an ankle or a wrist of a wearer, an electronic unit housed in the casing and a battery housed in the casing, the casing is made of at least one first part and one second part connected to each other by at least one mechanical connection, said mechanical connection comprising at least one pin extending from a first end face of the first part and at least one housing made opening in a first end face of the second part, the housing being configured to house the pin, the mechanical connection also comprising locking means to retain the at least one pin in the housing, said locking means comprising at least one locking plate movable transversely with respect to the housing axis and a drive unit for moving the locking plate, the locking plate and the pin being shaped in such manner that, in a locking state, the pin is retained in the housing.

The drive unit is preferably a non-reversible mechanism.

In one embodiment, the drive unit is controlled by the control unit, and comprises an electric motor powered by the battery and mechanical transmission means to move the locking plate.

For example, the electric motor is a rotative motor, the output shaft of which extending in a transversal direction with respect to the housing axis and the mechanical transmission means comprise an angle transmission connecting the output shaft and the locking plate.

In a preferred example, the drive unit comprises a cam driven in rotation by the electric motor, said cam cooperating with a cam edge made in the locking plate.

The angle transmission can comprise a drive gear connected directly to an output shaft of the electric motor, and a driven gear meshing the drive gear, and the cam being secured to the driven gear.

In a preferred embodiment, the cam comprises a radial protrusion and the cam edge comprises a notch configured to house the radial protrusion in a locking state.

The drive unit is advantageously configured to move the cam in a rest position in such manner that the cam does not contact the locking plate.

For example, the at least one pin comprises a groove surrounding at least partially the pin axis and the locking plate comprises a locking edge configured to enter into the groove in a locking state.

In a preferred example, the locking edge and the cam edge belong to a same recess.

The electronic monitoring bracelet can comprise a position sensor of the locking plate, for example a Hall sensor fixed to the casing and a magnet secured to the locking plate or to a part moving together with the locking plate.

According to an additional feature, the electronic monitoring bracelet comprises return means for making the locking plate return in an unlocking state.

Preferably, the locking plate abuts against the casing of the second part along a moving direction in the locking state.

In a preferred example, the first part comprises two pins extending from the first end face of the first part and the second part comprises two housings opening in the first end face of the second part, the locking plate being shaped for retaining each pin in its housing in a locking state.

The first part advantageously comprises a second end face and the second part comprises a second end face, both second end faces being connected by a hinge, advantageously a dismountable hinge.

### SHORT DESCRIPTION OF THE DRAWINGS

The present invention will be better understood with the following description and the appended drawings on which:
- Figure 1 is a perspective outer view of an electronic monitoring bracelet according to an example embodiment,
- Figure 2 is a perspective view of the electronic monitoring bracelet of fig-ure 1 showing the detail of the mechanical connection between the end faces of the parts of the bracelet,
- Figure 3 is a perspective view of the mechanical connection of figure 2, the casing being partially illustrated, in an unlocked state,
- Figure 4 is a sectional view of figure 3 along plane A,
- Figure 5 is a perspective view of the mechanical connection of figure 2, the casing being partially illustrated, in a rest state,
- Figure 6 is a sectional view of figure 5 along plane B, the casing being added and the pin being removed,
- Figure 7 is a perspective view of the mechanical connection of figure 2, the casing being partially illustrated, in a locking state,
- Figure 8 is a sectional view of figure 7 along plane C, the casing being added and the pins being removed.

### DETAILLED DESCRIPTION OF EMBODIMENTS

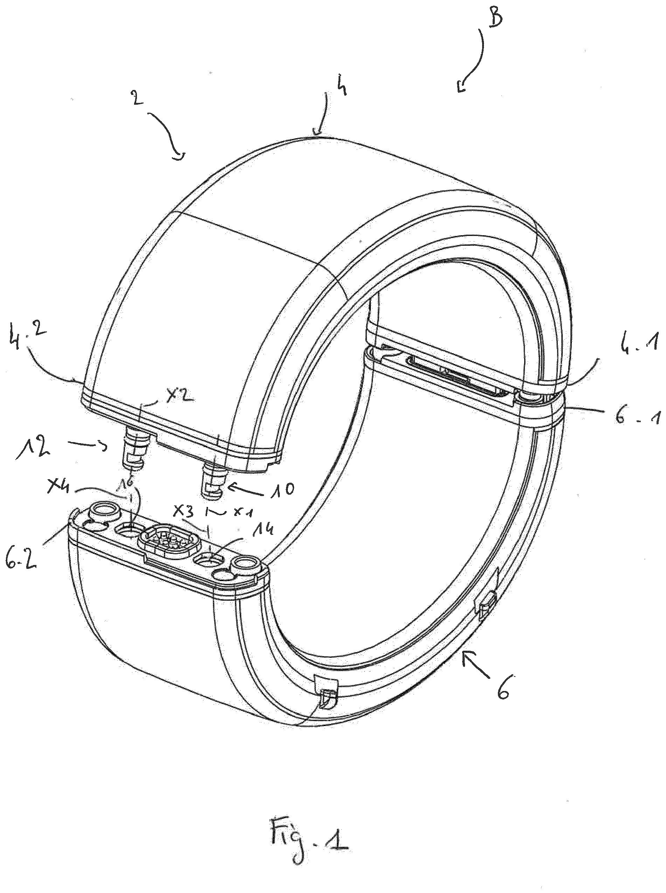

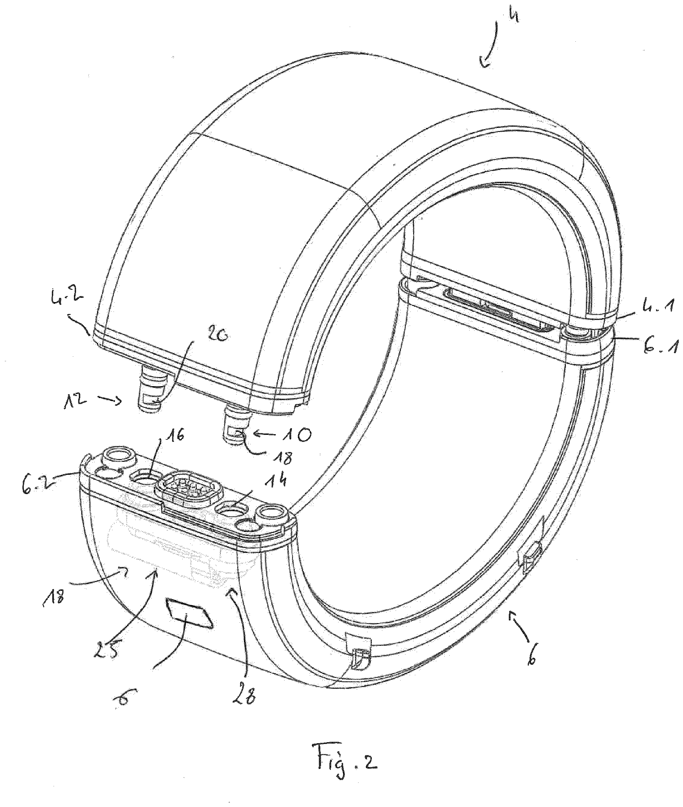

Figures 1 to 8 show an example embodiment of an electronic monitoring bracelet B according to the invention.

In this example embodiment, the electronic monitoring bracelet B, which will be designated by bracelet B in the following description, comprises a rigid ring-shape shell 2 made of several parts 4, 6, preferably two, which are configured to be rigidly connected to each other in order to surround the wrist or the ankle of a wearer without possibility for the wearer to take off the bracelet by making it slide along the ankle or the wrist or by separating both parts from each other. Both parts of the bracelet are half of a circle-shaped attached to each other by a mechanical connection including locking means that will be described in detail below. Locking means can be unlocked by specific control operations and are safe enough to prevent the wearer to take off the bracelet without authorization.

Electronic systems 6 (schematically represented) are located inside the shell to localize the wearer and monitor his/her displacements, for example they comprise satellite localization system. In addition, electronic systems comprise means to prevent the wearer from taking off the bracelet without this being detected.

The shell of the bracelet is for example made in a rigid plastic material.

Each portion 4, 6 comprises two end faces 4.1, 4.2, 6.1, 6.2 respectively.

In this example, the mechanical connection comprises a hinge between end faces 4.1 and 6.1.

Preferably, the hinge is a demountable one. In an unlocked state of the bracelet, both parts can be separated entirely and the risk of damage for the bracelet is reduced. The demountable hinge comprises for example legs on ends 4.1, 6.1 which cooperate with recesses in end faces 6.1, 4.1 In such a manner, rotative motion between parts 4 and 6 is ensured and can be easily detached from each other.

A bracelet with a non-demountable hinge belongs to the invention.

The mechanical connection comprises two pins 10, 12 projecting from the end face 4.2 in the circumferential direction; two housings 14, 16 opening in the end face 6.2 each housing being configured to house a pin 10, 12 respectively. The mechanical connection also comprises locking means 18 for locking each pin in the corresponding housing. The invention is not limited to two pins, a mechanical connection with one pin or more than two pins belongs to the invention.

Each pin 10, 12 extends along a longitudinal axis X1, X2 respectively.

Each housing 14, 16 extends along a longitudinal axis X3, X4 respectively.

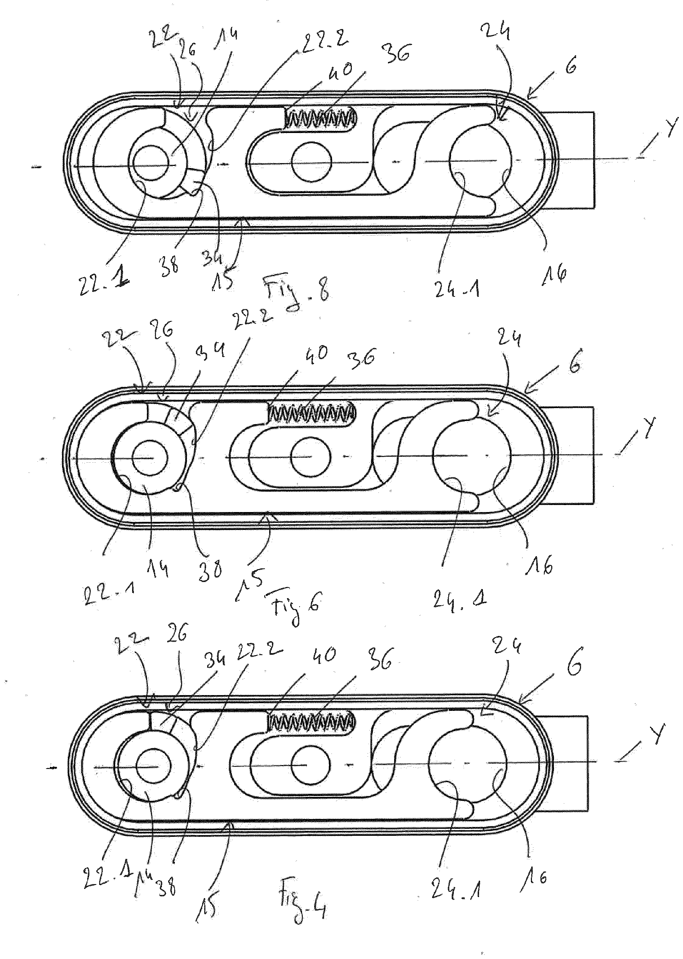

In the example shown on figures 4, 6 et 8, the cross-section of the housing 16 is oblong. As a variant, the cross-section of the housing 16 is circular.

When pin 10 is in housing 14 axis X1 et X3 are aligned.

When pin 12 is in housing 16 axis X2 et X4 are aligned.

The locking means are located at the closed end of the housings 14, 16.

The locking means comprise a locking plate 15 which is movable along a direction Y perpendicular to axis X3, X4 of the housings and a drive unit D to move the movable plate 15.

Each pin comprises a groove 18, 20 and the plate is shaped to cooperate with grooves 18, 20 in such manner to form an abutment for pins 10, 12 along X3, X4 axis. In this example, the locking plate comprises two recesses 22, 24.

In the illustrated example, the groove surrounds partially the pin axis which increases the material amount and the mechanical resistance. As a variant, the grooves are annular and surrounded the axis' entirely.

Each recess comprises a locking edge 22.1, 24.1 configured to enter into the groove 18, 20. The walls of the housings are constructed to allow the locking plate to cooperate with the pin grooves. As a variant, the locking plate comprises two holes instead of recess', each locking edge is then a part of the edge of the holes.

In the illustrated example, each recess 22, 24 has approximatively a C-shaped opening along the Y direction, the shape of the locking edge being approximatively an inner curvature of the C.

When the locking plate 15 moves to the left in the illustrated example, the locking edges enter into the grooves 18, 20 of the pins, and when the locking plate 15 moves to the right in the illustrated example, the locking edges 22.1, 24.1 move away from the grooves of the pins

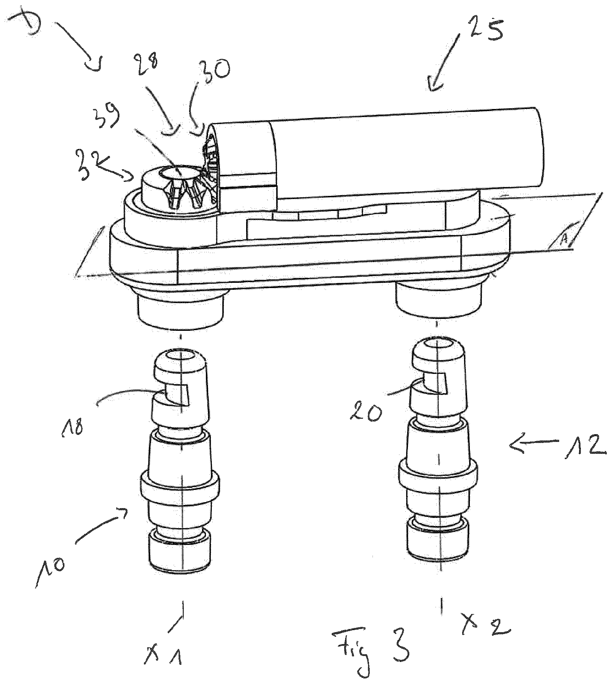

In an advantageous embodiment, the drive unit is a non-reversible drive unit. In figure 3, the non-reversible drive unit comprises an electric motor 25 and a cam 26 cooperating with a cam edge 22.2 of one recess 22.

The cam is driven in rotation by the electric motor 25. The drive unit is powered by a battery embedded in the casing. The drive unit is also connected to the control unit of the bracelet which controls the operation of the locking means.

In a preferred embodiment, the electric motor 25 is a rotating electric motor and is connected to the cam through an angle transmission 28. Thanks to this arrangement, the electric motor 25 is orientated perpendicularly to axis X3, X4 and parallel to the locking plate 15. This arrangement allows to reduce the footprint of the locking means.

In this example, free end of pin 10 passes through the cam and is housed in a bore made in the driven gear 32. This arrangement is very compact.

As a variant, the cam edge 22.2 and the locking edge do not belong to the same recess or the same hole. The free end of pin 10 may then be not housed in the driven gear.

The angle transmission 28 comprises a driving gear 30 fixed on the output shaft of the electric motor and a driven gear 32 meshing the driving gear 30. The cam 26 and the driven gear 32 are secured to each other such that the rotation of the driven gear rotates the cam 26. Preferably cam 26 and driven gear 32 are in in one piece.

Any other angle transmission can be provided.

Cam 26 comprises a radial protrusion 34 which contacts the cam edge 22.2 of the recess 22. The profile of the came edge 22.2 is chosen to ensure a safe blocking of the locking plate in locking state.

Elastic means 36 between the locking plate and the casing helps the locking plate to go back in an unlocking position. In the illustrated example, the elastic means 36 comprise a helicoidal spring mounted between a face 40 of the locking plate and the casing. Face 40 is advantageously provided in the middle part of the locking plate between both recesses, which results in a downsizing of the bracelet.

In a very advantageous manner, the cam edge 22.2 comprises a notch 38 at an end of the cam edge in which the cam protrusion is housed in a locking state. The notch 38 enhances the holding of the locking plate in locking position.

In a preferred embodiment, the casing is such that the locking plate abuts a casing portion in a separation direction of the parts 2, 4, improving the pull-out resistance.

Very advantageously, in a locking position, the locking plate 15 abuts a part of the casing of the second part 6 in the locking direction along the Y axis, to prevent the locking protrusion from escaping the notch 38 even when vibration is applied to the bracelet. A play is provided to allow the radial protrusion to escape the notch when the cam is rotated by the electric motor.

In a preferred embodiment, a position sensor is provided to know the position of the cam, and then the state of the bracelet. For example, the position sensor comprises a magnet 39 and a Hall sensor (not shown). The magnet is attached to the locking plate or to any piece moving with the locking plate. For example, the magnet 39 is embedded in the driven gear 32 and the Hall sensor is mounted on the casing close to the magnet to sense the rotation of the magnet. Any other type of position sensor, for example a contact sensor or an optical sensor can be provided.

An example of the operation of the locking means will be described below.

The bracelet is in an unlocking state. The locking plate is in the position shown on figures 3 and 4. The pins can slide axially in the housing in a free manner.

An instruction is sent to the drive unit to lock the bracelet.

The electric motor is powered and the drive gear rotates, which rotates the driven gear and the cam.

The radial protrusion 34 of cam 26 contacts the cam edge 22.2 of the recess 22. Due to the profile of the cam edge, the cooperation between the radial protrusion and the cam edge 22.2 triggers the sliding of the locking plate along the Y direction such that the locking edge of the recesses moves toward the pin's grooves.

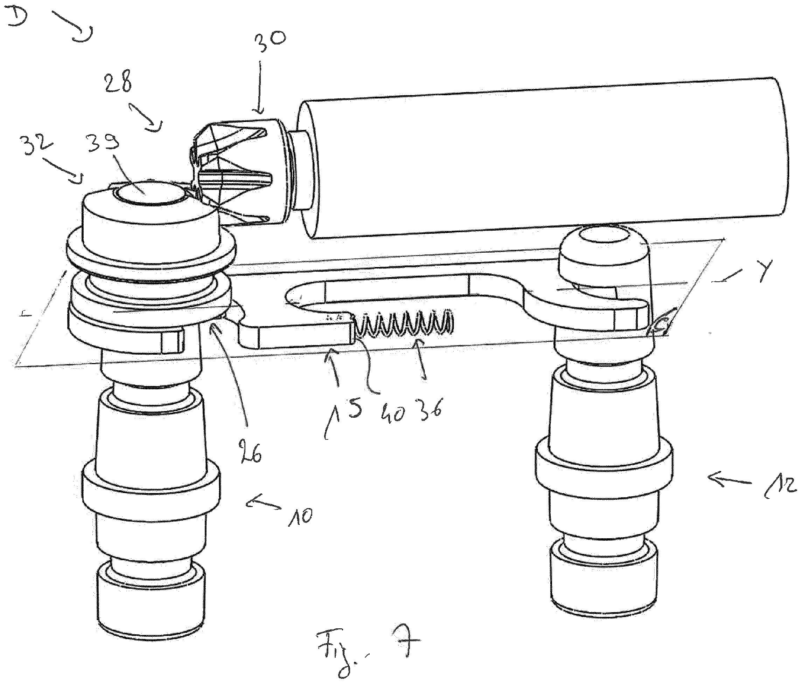

The cam rotates until the cam protrusion 34 enters into the notch 38 of the recess 22. In this position, the pins are axially retained in the housing through the abutment of the grooves against the locking plate (figures 7 and 8). The radial protrusion in the notch and the non-reversibility of the drive unit reduce the risk of uncontrolled unlocking.

For unlocking the bracelet, instructions are sent to the drive unit to rotate the cam in the counter-direction, the radial protrusion escapes the notch and thanks to the spring, the locking plate is pushed back in the unlocking position.

The recesses 22, 24 are disengaged from the pins' grooves. The pins can slide freely in the housings and both parts of the bracelet can be separated.

In this embodiment, the locking phase is active and the beginning of the unlocking phase is active as, in a first step, some power is needed to turn the cam in open position, and in a second step, the motion of the unlocking plate is carried out by the action of the return spring.

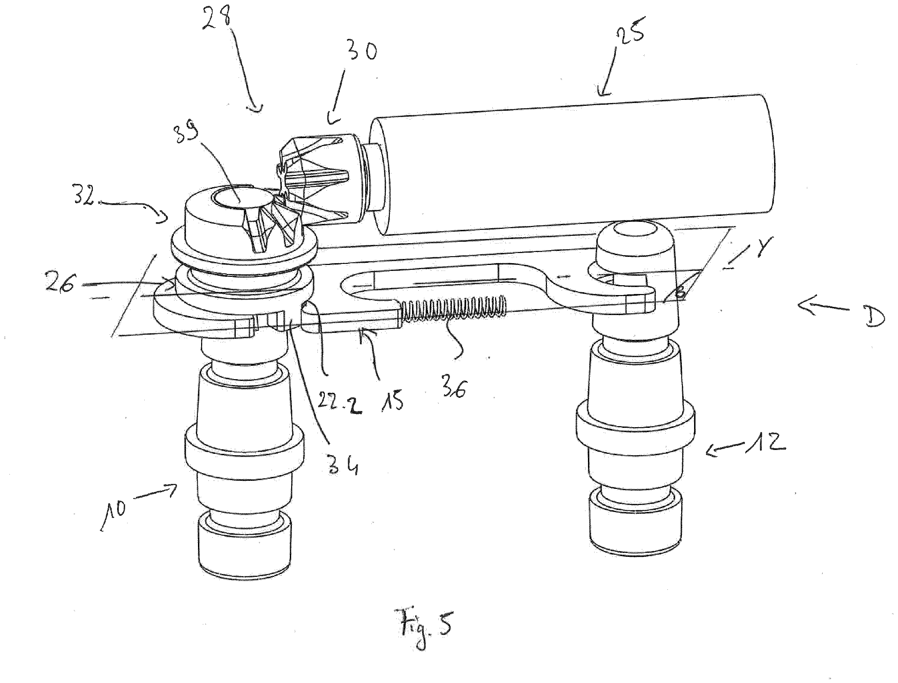

In a preferred embodiment, the cam is rotated until the radial protrusion abuts the locking plate and push back the locking plate. And then the cam is rotated in the first direction until the radial protrusion does not contact the locking plate anymore, which ensures that the cam is not damaged due to a motion of the locking plate (figures 5 and 6).

In the illustrated example, the locking plate is hollowed in its central part to reduce its weigh and the overall weight of the device. A locking plate with a plain central part belongs to the scope of the invention.

In another example, the drive unit comprises a rack on the locking plate meshing a gear driven by the electric motor.

The use of a single locking plate is very advantageous in reducing the cumbersome of the locking means. As a variant, the locking means comprises as many locking plates operated by one electric motor as the number of pins; each locking plate forming an axial abutment for a pin.

In another embodiment, the locking of the bracelet is obtained in a passive manner. For example, the spring is not a return spring but a spring which forces the locking plate in a prelocking position. In this prelocking position, the pins can slide through the recesses, and the locking edge are pushes back in the grooves. The drive unit is then actuated to rotate the cam to place the radial protrusion in the notch and confirm the locking. The unlocking is carried out by moving the locking plate such that the pins are released from the locking plate recesses. When the pins are slide out, the locking plate is moved back in the pre-locking position ready for the next connection. In this embodiment, the locking phase is passive and then active, and the unlocking phase is active.

As a variant, the pins comprise a free end with a diameter bigger than the diameter of the rest of the pin, and the locking edges form an abutment the free end.

As a variant, the hinge is replaced by at least one pin, a housing and locking means.

The invention offers an electronic monitoring bracelet which does not require electromagnets for locking and/or ulocking the several parts.

## Claims

1. Electronic monitoring bracelet (B) comprising a casing configured to surround an ankle or a wrist of a wearer, an electronic unit housed in the casing and a battery housed in the casing, the casing is made of at least one first part and one second part connected to each other by at least one mechanical connection, said mechanical connection comprising at least one pin (10, 12) extending from a first end face of the first part and at least one housing made opening in a first end face of the second part, the housing being configured to house the pin (10, 12), the mechanical connection also comprising locking means to retain the at least one pin (10, 12) in the housing, said locking means comprising at least one locking plate (15) movable transversely with respect to the housing axis and a drive unit for moving the locking plate (15), the locking plate (15) and the pin (10, 12) being shaped in such manner that, in a locking state, the pin (10, 12) is retained in the housing.

2. Electronic monitoring bracelet according to claim 1, in which the drive unit is a non-reversible mechanism.

3. Electronic monitoring bracelet according to claim 1 or 2, in which the drive unit is controlled by the control unit, and comprises an electric motor (25) powered by the battery and mechanical transmission means to move the locking plate (15).

4. Electronic monitoring bracelet according to claim 3, in which the electric motor (25) is a rotative motor, the output shaft of which extending in a transversal direction with respect to the housing axis and in which the mechanical transmission means comprise an angle transmission (28) connecting the output shaft and the locking plate (15).

5. Electronic monitoring bracelet according to claim 3 or 4, in which the drive unit comprises a cam (26) driven in rotation by the electric motor (25), said cam (26) cooperating with a cam edge (22.2) made in the locking plate (15).

6. Electronic monitoring bracelet according to claim 5 in combination with claim 4, in which the angle transmission comprises a drive gear (30) connected directly to an output shaft of the electric motor (25), and a driven gear (32) meshing the drive gear (30), and in which the cam (26) is secured to the driven gear (32).

7. Electronic monitoring bracelet according to claim 5 or 6, in which the cam (26) comprises a radial protrusion (34) and the cam edge (22.2) comprises a notch (38) configured to house the radial protrusion (34) in a locking state.

8. Electronic monitoring bracelet according to one of the claims 5 to 7, in which the drive unit is configured to move the cam (26) in a rest position in such manner that the cam does not contact the locking plate (15).

9. Electronic monitoring bracelet according to one of the claims 1 to 8, in which the at least one pin (10, 12) comprises a groove (18, 20) surrounding at least partially the pin (10, 12) axis and the locking plate (15) comprises a locking edge configured to enter into the groove in a locking state.

10. Electronic monitoring bracelet according to claim 9 in combination with one of the claims 5 to 8, in which the locking edge (22.1) and the cam edge (22.2) belong to a same recess (22).

11. Electronic monitoring bracelet according to one of the preceding claims, comprising a position sensor of the locking plate, for example a Hall sensor fixed to the casing and a magnet secured to the locking plate or to a part moving together with the locking plate.

12. Electronic monitoring bracelet according to one of the preceding claims, comprising return means (36) for making the locking plate return in an unlocking state.

13. Electronic monitoring bracelet according to one of the preceding claims, in which the locking plate (15) abuts against the casing of the second part along a moving direction in the locking state

14. Electronic monitoring bracelet according to one of the preceding claims, in which the first part comprises two pins extending from the first end face of the first part and the second part comprises two housings opening in the first end face of the second part, the locking plate being shaped for retaining each pin in its housing in a locking state.

15. Electronic monitoring bracelet according to the preceding claims, in which the first part comprises a second end face and the second part comprises a second end face, both second end faces being connected by a hinge, advantageously a dismountable hinge.
